# EUROPÄISCHE PATENTSCHRIFT

(11) **EP 0 472 017 B1**
(45) Veröffentlichungstag und Bekanntmachung des Hinweises auf die Patenterteilung: **21.09.1994**
(21) Anmeldenummer: 91112853.6
(22) Anmeldetag: 31.07.1991
(51) Int. Cl.: A61B 17/58, F16B 25/00

(54) **Zylindrischer Körper mit an der Aussenfläche ausgeformtem Gewinde**
Cylindrical bodies with external thread
Corps cylindriques filetés

(30) Priorität: 24.08.1990 DE 4026777
(43) Veröffentlichungstag der Anmeldung: 26.02.1992
(73) Patentinhaber: Härle, Anton, Prof. Dr., D-48161 Münster (DE)
(72) Erfinder: Härle, Anton, Prof. Dr., D-48161 Münster (DE)
(74) Vertreter: Habbel, Hans-Georg, Dipl.-Ing.

(56) Entgegenhaltungen:
- WO-A-89/00028
- WO-A-90/02526
- FR-A- 2 401 349
- FR-A- 2 559 378
- US-A- 3 752 030
- Handbook Tool Engineers (New York, 1959 Seiten 46/33 und 46/34 undVerkaufskatalog der Firma Würth (Seite 44)

## Beschreibung

Die Erfindung bezieht sich auf einen Satz zylindrischer Schrauben mit unterschiedlichen Außendurchmessern gemäß dem Oberbegriff des Hauptanspruches.

Eine in Form einer Schraubenlinie in die Außenfläche eines zylindrischen Körpers eingeschnittene Nut von bestimmtem Profil wird in der Technik als Gewinde bezeichnet. Diese Nut ermöglicht, eine drehende Bewegung des zylindrischen Körpers in eine Längsbewegung umzuwandeln. Das Maßsystem für Gewinde baut auf Außendurchmesser und zugehöriger Steigung auf.

Aus dem "Handbook Tool Engineers" (Neuhook 1959, Seiten 46/33 und 46/34) ist ein Satz gewindeherstellender Werkzeuge bekannt, die sich durch kleine Beträge des Außendurchmessers unterscheiden und durch ihre aufeinanderfolgende Anwendung zur Herstellung eines Gewindes geeignet sind. Den einzelnen Werkzeugen dieses Werkzeugsatzes ist keine eigene unterschiedliche Schraubengröße zugeordnet.

Aus dem Verkaufskatalog der Firma Würth (Seite 44, veröffenlicht 1987) sind zahlreiche Sortimente von zylindrischen Schrauben mit unterschiedlichen Außendurchmessern bekannt, wobei die Schrauben ein in Form einer Schraubenlinie in der Außenfläche ausgeformtes gewinde aufweisen.

Nachfolgend wird am Beispiel eines Knochenschrauben- und/oder Gewindebohrersatzes für Osteosynthesearbeiten die der Erfindung zugrundeliegende Überlegung erläutert.

Knochenschrauben bzw. Stifte mit Knochengewinde-Anteil werden meist in Kombination mit Platten- und Stabsystemen angewandt, um Knochen bzw. Knochenteile in einer bestimmten Stellung und Ausrichtung zueinander zu fixieren.

Bei der herkömmlichen Osteosynthese an Röhrenknochen wird dabei eine mit Löchern versehene Platte mittels durch diese Löcher hindurchgreifender Knochenschrauben am Knochen fixiert. Die Ruhigstellung der Knochen bzw. Knochenteile erfolgt durch Anpressen an die Platte mittels Schrauben, wobei die Schraubenköpfe auf der dem Knochen gegenüberliegenden Seite in den angeschrägten Schraubenlöchern ein Widerlager finden. Die Knochenschrauben selbst finden ihre Verankerung in Knochen-Bohrkanälen, die mit auf die Größenverhältnisse abgestimmten Bohrern hergestellt und in denen wiederum mit entsprechend dimensionierten Gewindebohrern ein Gewinde eingeschnitten worden ist (US-A-4943 292).

Den größten Teil des Halts finden die Knochenschrauben dabei in der kortikalen Knochenrinde, während in der Spongiosa und in der Markhöhle kein wesentlicher Widerhalt zu erreichen ist. Die im Knochen verankerten Knochenschrauben werden in ihrem Verlauf im Knochen selbst praktisch nur auf Zug beansprucht.

Die Stabilität einer solchen Osteosynthese wird damit hauptsächlich durch die Ausrißfestigkeit der Knochenschrauben aus dem Knochen bestimmt. Wird beim Anziehen der Schraube das Gewinde im Knochen überlastet, d. h. durchgedreht, ist der größte Teil der Stabilität verloren. Hilfsweise werden nun Schraubenmuttern an der plattenabseitigen Knochenseite auf eine längere Schraube aufgedreht und so eine gewisse Stabilität hergestellt.

Bei Verplattungsverfahren an der Wirbelsäule ist die stabile Verankerung der Knochenschrauben im Knochen noch problematischer als bei der Bearbeitung normaler Röhrenknochen, da aufgrund der anatomischen Verhältnisse jeder Knochen nur durch eine Knochenschraube an der Knochenplatte fixiert werden kann. Da einerseits erhebliche Kräfte und Belastungen auftreten - nahezu das ganze Körpergewicht lastet auf einer fixierenden Schraube - kann eine für die knöcherne Konsolidierung erforderliche Ruhigstellung nur schwer erreicht werden. Die Auslockerung wiederum hat ihre Ursache darin, daß eine solide Verankerung nur im Kontaktbereich der Knochenschraube mit kortikalen Strukturen gegeben ist (DE-A-3639 522).

Während bei den Röhrenknochen der Extremitäten die Schraube immer in zwei Knochenrinden verankert wird, werden bei der Wirbelsäulenfixation die Schrauben durch die engen Knochenverbindungen zwischen dem vorn liegenden Wirbelkörper und dem hinten liegenden Wirbelbogen eingedreht. Diese Knochenbrücken - Bogenwurzeln oder Pedikel genannt - haben im sagittalen Schnitt die Form einer Zwirnspule, wobei nur im mittleren, d. h. dem engen Abschnitt, eine gute, direkte Kraftübertragung auf die zentral verlaufende und nur hier mit der Knochenrinde tangentialen Kontakt aufnehmende Schraube erfolgen kann.

Im frontalen Querschnitt sind diese Pedikel längs-oval und in den verschiedenen Wirbelsäulenabschnitten sehr unterschiedlich gestaltet. Eine stabile Fixation ist an der Wirbelsäule daher schon primär schwierig und nur dann möglich, wenn das Gewinde der Knochenschraube mit der Pedikel-Kortikalis im Isthmusbereich direkten Kontakt aufnimmt. Außerdem kann der Ort des maximalen Durchmessers bei der Herstellung des Knochenkanales selten ausfindig gemacht werden, so daß die Dimensionierung der zu wählenden Gewindebohrer und Knochenschrauben in jedem Fall unbekannt ist. Da unmittelbar neben den Pedikeln die Nervenwurzeln und das Rückenmark liegen, dürfen die Schrauben nicht den Pedikel-Isthmus verlassen.

Erschwerend kommt noch hinzu, daß die frontalen Durchmesser der Pedikel eine große Variabilität aufweisen und bei jedem Segment zwischen Minimal- und Maximalwert rund 6 mm liegen. Eine stabile Verankerung einer Knochenschraube setzt aber eine auf den gegebenen Durchmesser des Knochenkanales abgestimmte Dimensionierung des Gewindebohrers und vor allem des Gewindepins voraus. Unterdimensionierung beinhaltet die Gefahr der Instabilität der Knochen-Schrauben-Verbindung und des Implantatabbruches. Überdimensionierung der Implantate kann leicht neurologische Komplikationen bis zu Querschnittslähmungen nach sich ziehen.

Der Erfindung liegt daher die Aufgabe zugrunde, eine Schraubenkonstruktion zu schaffen, die bei ausgelockertem Gewinde bei gleicher Gewindecharakteristik einen neuen Festsitz schafft und einen Gewindebohrer zu schaffen, der ein neues Gewinde nachschneidet bei optimaler Verankerung der einzusetzenden Schraube.

Diese der Erfindung zugrundeliegende Aufgabe wird durch die Lehre des Hauptanspruches gelöst.

Die Erfindung ist damit einsetzbar bei Osteosynthesearbeiten und bei in Holz, Kunststoff oder weichen Metallen einzusetzenden Schrauben.

Mit anderen Worten ausgedrückt wird ein Satz von gewindeherstellenden Werkzeugen für Osteosynthesearbeiten mit ein Satz von Schrauben geschaffen, wobei aber die Gewinde der gewindeherstellenden Werkzeuge und der Schrauben bzw. mit Gewinde versehenen Knochenpins unabhängig von ihrem unterschiedlichen Außendurchmesser so gestaltet sind, daß sie gleiche Steigung aufweisen.

Somit ist es möglich, mit einem Satz von gewindeherstellenden Werkzeugen zu arbeiten, die sich durch kleine Beträge des Außendurchmessers unterscheiden, die eine solche Gewindeausgestaltung aufweisen, daß bei Anwendung des nächstgrößeren Werkzeuges eine Beschädigung des durch das vorher eingesetzte Werkzeug geschnittenen Gewindes nicht erfolgt.

Nunmehr ist es bei Osteosynthesearbeiten möglich, bei Handhabung z. B. des Gewindebohrers den Widerstand abzutasten, der sich dem Gewindebohrer stellt, so daß von Hand abgetastet werden kann, wann sich die äußeren Gewindegänge in der kortikalen Knochenrinde befinden. Das Gewinde wird also so lange eingeschnitten, bis eine feste Verankerung in der Pedikel-Kortikalis erreicht wird. Dann erfolgt das Eindrehen einer zu diesem Querschnitt passenden Knochenschraube.

Beim Auslockern einer Schraube kann mit dem gewindeherstellenden Werkzeugsatz ein neues, einen festen Halt verschaffendes Gewinde nachgeschnitten werden und eine im Durchmesser größere Schraube eingesetzt werden. Entscheidend bei diesem Vorgang ist, daß durch das mehrmalige Anwenden z. B. des Gewindebohrers das Gewinde nicht beschädigt wird.

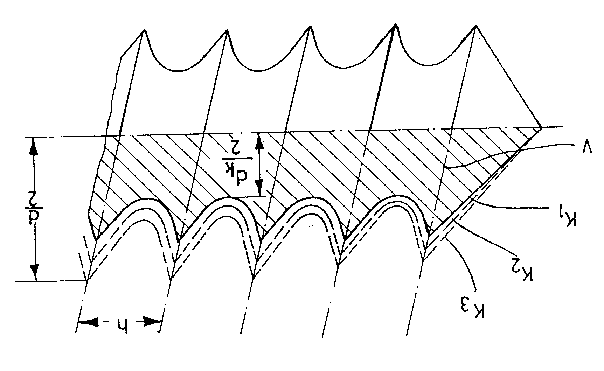

Ein Ausführungsbeispiel der Erfindung wird nachfolgend anhand der Zeichnung erläutert.

In der Zeichnung ist schematisch die Gewindeausbildung von drei unterschiedliche Außendurchmesser aufweisenden Schraubenteilen dargestellt. In der Zeichnung sind dabei die verschiedenen Schraubenteile mit K₁, K₂ und K₃ bezeichnet. Die Steigung ist mit dem Zeichen h versehen, der Außendurchmeser mit dem Zeichen d und der Kerndurchmesser mit der Bezeichnung d_{K}.

Die Gewindegänge der drei Teilschrauben sind übereinander dargestellt und in der Zeichnung sind die Gewindescheitel der drei Teilschrauben K₁, K₂ und K₃ durch eine Verbindungslinie V verbunden. Diese Verbindungslinie liegt bei der erfindungsgemäßen Ausbildung auf der Winkelhalbierenden der Gewindegänge der drei Schrauben K₁, K₂ und K₃.

Durch eine solche Gestaltung wird erreicht, daß bei Anwenden aufeinanderfolgender Gewindebohrer bzw. aufeinanderfolgender Schrauben ein Verletzen der vorher vorhandenen Gewindegänge nicht eintritt.

## Patentansprüche

1. Satz zylindrischer Schrauben mit unterschiedlichen Außendurchmessern, wobei die Schrauben ein in Form einer Schraubenlinie in der Außenfläche ausgeformtes Gewinde aufweisen, dadurch gekennzeichnet, daß trotz unterschiedlicher Außendurchmesser (d) der Schraube, das Gewinde jeder Schraube gleiche Steigung (h) besitzt, wobei sich die Außendurchmesser der Schrauben durch derart kleine Beträge unterscheiden, daß bei Anwenden aufeinanderfolgender Schrauben ein Verletzen der vorher vorhandenen Gewindegänge nicht eintritt.

2. Satz zylindrischer Schrauben nach Anspruch 1, dadurch gekennzeichnet, daß das Gewinde auch bei unterschiedlichen Kerndurchmessern d_{K}) gleiche Steigung (h) besitzt.

3. Satz zylindrischer Schrauben nach Anspruch 1, dadurch gekennzeichnet, daß die Verbindungslinie (V) durch die Gewindescheitel der unterschiedliche Außendurchmesser (d) aufweisenden Schrauben die Winkelhalbierende des Gewindeganges ist.

4. Satz zylindrischer Schrauben nach Anspruch 1 oder 2, dadurch gekennzeichnet, daß der Außendurchmesser der unterschiedlichen Schrauben um mindestens 0,05 mm differiert.

5. Satz zylindrischer Schrauben nach einem der vorhergehenden Ansprüche, dadurch gekennzeichnet, daß der Durchmesser der ersten Gewindegänge dem der Gewindegänge der nächstkleineren Schrauben angenähert ist und sich dann vergrößert.

6. Satz zylindrischer Schrauben nach einem der vorhergehenden Ansprüche, dadurch gekennzeichnet, daß die Kerndurchmesser gegenüber dem zugehörigen Außendurchmesser überproportional ansteigen.

7. Satz zylindrischer Schrauben nach einem der vorhergehenden Ansprüche, dadurch gekennzeichnet, daß die Schrauben als Knochenschraubensatz für Osteosynthesearbeiten ausgebildet sind.

8. Satz zylindrischer Schrauben nach einem der vorhergehenden Ansprüche, dadurch gekennzeichnet, daß jeder Schraubengröße ein Gewindebohrer zugeordnet ist, wobei die Gewindebohrer ein in Form einer Schraubenlinie in der Außenfläche ausgeformtes Gewinde aufweisen, und daß trotz unterschiedlicher Außendurchmesser (d) der Gewindebohrer, das Gewinde eines jeden Gewindebohrers die gleiche Steigung (h) besitzt.

9. Satz zylindrischer Schrauben nach Anspruch 8, dadurch gekennzeichnet, daß die Gewindebohrer für Osteosynthesearbeiten ausgebildet sind.

10. Satz zylindrischer Schrauben nach den Ansprüchen 7 und 9, dadurch gekennzeichnet, daß die Außen- und Kerndurchmesser der Knochenschrauben 0,05 bis 0,2 mm über dem Außen- und Kerndurchmesser der entsprechenden Gewindebohrer liegen.

11. Satz zylindrischer Schrauben nach einem der vorhergehenden Ansprüche, dadurch gekennzeichnet, daß jeder Schraubengröße ein Schraubenschneider- oder Rollersatz zugeordnet ist.

12. Satz zylindrischer Schrauben nach einem der vorhergehenden Ansprüche, dadurch gekennzeichnet, daß jeder gewindeherstellenden Werkzeuggröße ein Werkzeug zur Herstellung dessen Gewindes zugeordnet ist.

## Claims

1. A set of cylindrical screws with various external diameters, the screws comprising a thread formed in the outer surface in the form of a helix, characterized in that, despite the varying external screw diameters (d), the thread of each screw has the same pitch (h), the external diameters of the screws differing by such small amounts that, if successive screws are used, damage to the previously present thread turns does not occur.

2. A set of cylindrical screws according to claim 1, characterized in that the thread also has the same pitch (h) with varying core diameters (d_{K}).

3. A set of cylindrical screws according to claim 1, characterized in that the connecting line (V) through the thread apexes of the screws of varying external diameters (d) is the angle bisecting line of the thread.

4. A set of cylindrical screws according to claim 1 or claim 2, characterized in that the external diameter of the various screws differs by at least 0.05 mm.

5. A set of cylindrical screws according to any one of the preceding claims, characterized in that the diameter of the first thread turns is close to that of the turns of the next-smaller screws and then gets bigger.

6. A set of cylindrical screws according to any one of the preceding claims, characterized in that the core diameters increase superproportionally with respect to the associated external diameter.

7. A set of cylindrical screws according to any one of the preceding claims, characterized in that the screws are constructed as a bone screw set for osteosynthesis work.

8. A set of cylindrical screws according to any one of the preceding claims, characterized in that a screw tap is associated with each screw size, the tap comprising a thread formed in the outer surface in the form of a helix and in that, despite the varying external tap diameters (d), the thread of each tap has the same pitch (h).

9. A set of cylindrical screws according to claim 8, characterized in that the taps are constructed for osteosynthesis work.

10. A set of cylindrical screws according to claims 7 and 9, characterized in that the external and core diameters of the bone screws are from 0.05 to 0.2 mm above the external and core diameters of the corresponding taps.

11. A set of cylindrical screws according to any one of the preceding claims, characterized in that a screw cutter or roller set is associated with each screw size.

12. A set of cylindrical screws according to any one of the preceding claims, characterized in that associated with each thread-producing tool size is a tool for producing the thread thereof.

## Revendications

1. Jeu de vis cylindriques ayant différents diamètres extérieurs, les vis comportant un filetage réalisé dans la surface extérieure sous forme d'une hélice, caractérisé en ce que, malgré un diamètre extérieur différent (d) de la vis, le filetage de chaque vis présente le même pas (h), les diamètres extérieurs des vis différant peu les uns des autres de façon que, lors de l'utilisation de vis successives, il ne se produise pas une détérioration des spires précédentes.

2. Jeu de vis cylindriques selon la revendication 1, caractérisé en ce que le filetage présente un pas constant (h) même en présence de diamètres différents à fond de filet (d_{K}).

3. Jeu de vis cylindriques selon la revendication 1, caractérisé en ce que la ligne (V), qui passe par les sommets des filetages des vis présentant des diamètres extérieurs différents (d), est la bissectrice de la spire.

4. Jeu de vis cylindriques selon la revendication 1 ou 2, caractérisé en ce que le diamètre extérieur des différentes vis varie d'au moins 0,05 mm d'une vis à l'autre.

5. Jeu de vis cylindriques selon l'une des revendications précédentes, caractérisé en ce que le diamètre des premières spires se rapproche de celui des spires de la vis plus petite immédiatement suivante, puis augmente.

6. Jeu de vis cylindriques selon l'une des revendications précédentes, caractérisé en ce que les diamètres à fond de filet augmentent d'une manière plus que proportionnelle par rapport au diamètre extérieur correspondant.

7. Jeu de vis cylindriques selon l'une des reendications précédentes, caractérisé en ce que les vis sont conçues comme un jeu de vis à os pour les opérations d'ostéosynthèse.

8. Jeu de vis cylindriques selon l'une des revendications précédentes, caractérisé en ce que, à chaque taille de vis, est affecté un taraud, les tarauds comportant un filetage constitué dans la surface extérieure sous forme d'une hélice, et en ce que, malgré des diamètres extérieurs (d) différents des tarauds, le filetage de chacun des tarauds a le même pas (h).

9. Jeu de vis cylindriques selon la revendication 8, caractérisé en ce que les tarauds sont destinés à des opérations d'ostéosynthèse.

10. Jeu de vis cylindriques selon l'une des revendications 7 et 9, caractérisé en ce que les diamètres extérieurs et les diamètres à fond de filet des vis à os sont de 0,05 à 0,2 mm supérieurs aux diamètres extérieurs et aux diamètres à fond de filet des tarauds correspondants.

11. Jeu de vis cylindriques selon l'une des revendications précédentes, caractérisé en ce que, à chaque taille de vis, est affecté un jeu d'outils à fileter ou un jeu de mollettes.

12. Jeu de vis cylindriques selon l'une des revendications précédentes, caractérisé en ce que, à chaque taille d'outil à fileter, est affecté un outil destiné à réaliser ce filetage.
